# EUROPÄISCHE PATENTANMELDUNG

(11) **EP 1 923 076 A1**
(43) Veröffentlichungstag der Anmeldung: **21.05.2008**
(21) Anmeldenummer: 06025397.8
(22) Anmeldetag: 08.12.2006
(51) Int. Cl.: A61L 9/12, A61L 9/14, A47F 3/00

(54) **Vorrichtung zur Abgabe von Duftstoffen**

(30) Priorität: 16.11.2006 EP 06023793
(71) Anmelder: Aldi Einkauf GmbH & Co. oHG, 45476 Mülheim/Ruhr (DE)
(72) Erfinder: Ochsenschläger, Robert, 45470 Mülheim/Ruhr (DE); Ernst, Peter, 45478 Mülheim/Ruhr (DE)
(74) Vertreter: Nunnenkamp, Jörg

(57) **Zusammenfassung**

Gegenstand der Erfindung ist eine Vorrichtung zur Abgabe von Duftstoffen (1) an beispielsweise Verkaufsräume (2), im Bereich von Warenpräsentationsmöbeln (3) oder dergleichen. Dazu verfügt die Vorrichtung über einen Duftstoffspender (4; 5, 6) und einen Duftstoffverteiler (7). Erfindungsgemäß wird eine an sich bekannte Luftumwälzeinrichtung (8) mit dem Duftstoffspender (4; 5, 6) ausgerüstet, so dass deren Luftumwälzeinheit (7) zugleich als Duftstoffverteiler (7) fungiert.

## Beschreibung

Die Erfindung betrifft eine Vorrichtung zur Abgabe von Duftstoffen an beispielsweise Verkaufsräume, im Bereich von Warenpräsentationsmöbeln oder dergleichen, mit einem Duftspender und einem Duftstoffverteiler.

Solche Vorrichtungen sind grundsätzlich bekannt und werden beispielsweise zur Verkaufsförderung eingesetzt, oder auch dazu, bestimmte Stimmungen bei visuellen und/oder akustischen Darbietungen zu unterstützen (vgl. DE 196 26 602 A1). Zu diesem Zweck schlägt die bekannte Lehre einen flachen plattenförmigen Grundkörper vor, welcher im Wesentlichen parallel zu seiner Ober- und Unterseite von mehreren separaten Kanälen durchsetzt ist. Die fraglichen Kanäle dienen zur Aufnahme der gasförmig abzugebenden Stoffe bzw. Duftstoffe und verfügen über je eine Ein- und Auslassöffnung. Die Kanäle werden von einem zugeführten Gasstrom durchströmt. Das hat sich grundsätzlich bewährt.

Darüber hinaus kennt man durch die DE 600 10 265 T2 eine Vorrichtung zur Abgabe von Duftstoffen, bei welcher eine erste Duftstoffzusammensetzung mit einer zweiten Duftstoffzusammensetzung gemischt wird, die periodisch zugeführt wird. Auch in diesem Fall ist eine gänzlich autark arbeitende Vorrichtung vorgesehen.

Der Stand der Technik hat sich insgesamt bewährt, was die grundsätzliche Abgabe von Duftstoffen in Räume angeht. Allerdings sind hierzu immer separat anzubringende Vorrichtungen erforderlich. Das ist aufwändig. Hier setzt die Erfindung ein.

Der Erfindung liegt das technische Problem zugrunde, eine derartige Vorrichtung so weiterzuentwickeln, dass der Aufwand und die Kosten verringert werden.

Zur Lösung dieser technischen Problemstellung schlägt die Erfindung bei einer gattungsgemäßen Vorrichtung vor, dass eine an sich bekannte und vorteilhaft im betreffenden Verkaufsraum bzw. im Bereich des Warenpräsentationsmöbels bereits vorhandene Luftumwälzeinrichtung mit dem Duftstoffspender ausgerüstet wird, so dass deren Luftumwälzeinheit nicht nur für die Luftumwälzung sorgt, sondern zugleich als Duftstoffverteiler fungiert.

Die Erfindung greift also ausdrücklich nicht auf eine gleichsam autarke Vorrichtung zur Abgabe von Duftstoffen zurück, sondern nutzt eine bereits vorhandene bzw. an sich bekannte Luftumwälzeinrichtung dazu, diese im Sinne einer zusätzlichen Duftabgabe aufzurüsten. Denn die fragliche Luftumwälzeinrichtung wird mit dem Duftstoffspender ausgerüstet.. Dadurch übernimmt die obligatorische Luftumwälzeinheit der Luftumwälzeinrichtung eine gleichsam doppelte Funktion. Sie sorgt einerseits im Rahmen ihrer originären Zweckbestimmung dafür, dass die Luft im Verkaufsraum bzw. im Bereich der jeweiligen Warenpräsentationseinheit bzw. des Warenpräsentationsmöbels umgewälzt wird. Andererseits wird die fragliche Luftumwälzung bzw. die Luftumwälzeinheit zugleich im Rahmen der Erfindung dafür genutzt, dass der Duftstoff verteilt wird. Das heißt, Luftumwälzeinheit und Duftstoffverteiler sind deckungsgleich.

Folgerichtig ist Gegenstand der Erfindung auch die Verwendung einer an sich bekannten Luftumwälzeinrichtung in Verbindung mit einem zusätzlichen Duftstoffspender zur gleichzeitigen Luftumwälzung und Luftbeduftung im Zuge der Verkaufsförderung. Dabei kann die gleichzeitige Luftumwälzung und Luftbeduftung in beispielsweise Räumen, insbesondere Verkaufsräumen, durchgeführt werden. Alternativ oder zusätzlich ist die Luftumwälzung/Luftbeduftung bei Warenpräsentationseinheiten bzw. Warenpräsentationsmöbeln denkbar.

Vorteilhaft ist die Luftumwälzeinrichtung als Kühleinrichtung oder Klimatisierungseinheit ausgebildet und/oder Bestandteil einer solchen Kühleinrichtung oder Klimatisierungseinheit. Meistens ist die Luftumwälzeinrichtung einem Warenpräsentationsmöbel zugeordnet und/oder in ein solches Warenpräsentationsmöbel integriert. Bei dem Warenpräsentationsmöbel handelt es sich vorteilhaft um ein Kühlmöbel, beispielsweise eine Kühltheke, einen Kühlschrank, ein Kühlregal etc..

Die Luftumwälzeinrichtung kann auch autark ausgebildet sein und beispielsweise als fahrbares Kühlmöbel mit einem Warenpräsentationsmöbel kombiniert werden oder allgemein im Verkaufsraum angeordnet werden. Anstelle eines autarken Kühlmöbels ist selbstverständlich auch ein autarkes Klimatisierungsmöbel denkbar. Außerdem kann die Luftumwälzeinrichtung zusammen mit dem Warenpräsentationsmöbel ein Aggregat aus den beiden vorerwähnten Elementen bilden. Dazu mögen die Luftumwälzeinrichtung und das Warenpräsentationsmöbel (lösbar) miteinander verbunden werden.

Im Detail hat es sich bewährt, wenn der Duftstoffspender eine Verteileinrichtung für den flüssigen Duftstoff, beispielsweise einen Spraykopf, eine Düse, einen heizbaren Docht etc. aufweist. Dann wird der Duftstoff von einem Reservoir ausgehend zu kleinen Tröpfchen fein verteilt in den von der Luftumwälzeinrichtung bzw. deren Luftumwälzeinheit zur Verfügung gestellten Luftstrom als gleichsam Aerosol abgegeben. Der Duftstoff als solcher mag in flüssiger oder auch fester Form vorliegen.

Als Alternative schlägt die Erfindung als Duftstoffspender ein Trägermaterial zur Aufnahme des Duftstoffes vor. Das Trägermaterial kann porös ausgeführt sein und beispielsweise eine Matte, einen Filter etc. darstellen. In den Poren des Trägermaterials findet sich der meistens flüssige Duftstoff. Das Trägermaterial wird im von der Luftumwälzeinheit erzeugten Luftstrom platziert, so dass der Duftstoff in Form von fein verteilten Tröpfchen mit dem Luftstrom mitgeführt wird. Dabei hat sich eine Anordnung des Duftstoffspenders im Bereich eines Luftauslasses der Luftumwälzeinrichtung bewährt.

Schließlich liegt es im Rahmen der Erfindung, den Duftstoffspender mit einer Befeuchtungseinheit zu kombinieren. In diesem Fall werden in den von der Luftumwälzeinheit erzeugten Luftstrom also nicht nur feinste Flüssigkeitspartikel des Duftstoffes, sondern auch feinste Wassertröpfchen abgegeben und in dem zu beaufschlagenden Raum, im Bereich der Warenpräsentationseinheit etc. abgegeben. Folglich wird dann ein gleichsam Luft-/Wassertropfen-/Duftstofftropfen-Aerosol gebildet.

Im Ergebnis wird eine Vorrichtung zur Abgabe von Duftstoffen im Rahmen der Erfindung vorgeschlagen, die auf eine ohnehin vorhandene oder beabsichtigte Luftumwälzeinrichtung in einem Raum zurückgreift und deren Luftumwälzeinheit zugleich als Duftstoffverteiler nutzt. Dabei kommt als Luftumwälzeinheit der an sich bekannten Luftumwälzeinrichtung nicht nur eine solche in Frage, die für die Bereitstellung von Zuluft, also bei einem Kühlmöbel von Kühlluft, eingesetzt wird. Sondern von der Luftumwälzeinheit kann im Rahmen der Erfindung und nach besonders bevorzugter Ausgestaltung auch Abluft bereitgestellt und für die Verteilung der Duftstoffe eingesetzt werden. Solche Abluft wird beispielsweise bei Kühlmöbeln als gleichsam Verlustluft in großer Menge in den zugehörigen Raum bzw. Verkaufsraum abgegeben und kann nun erfindungsgemäß dazu genutzt werden, gleichzeitig für die Verteilung des Duftstoffes zu sorgen.

Das heißt die Zuluft und/oder Abluft der Luftumwälzeinrichtung wird als Träger für den Duftstoff genutzt. Anders ausgedrückt, kann die Luftumwälzeinheit als Zuluftverteiler und/oder Abluftverteiler ausgeführt sein. In beiden Fällen fungiert der betreffende Verteiler zugleich als Duftstoffverteiler. Es handelt sich folglich um einen Zuluft-/Duftstoffverteiler und/oder Abluft-/Duftstoffverteiler.

Dabei versteht es sich, dass der Duftstoffspender kontinuierlich oder diskontinuierlich, beispielsweise in bestimmten Zeitabständen getaktet betrieben werden kann. Dadurch lässt sich die Konzentration des Duftstoffes bzw. von mehreren Duftstoffen in dem Raum, insbesondere Verkaufsraum, gezielt variieren und für die entsprechende Verkaufsförderung nutzen. Denkbar ist es an dieser Stelle, die Konzentration des einen Duftstoffes bzw. der mehreren Duftstoffe im zugehörigen Raum mit Hilfe eines oder mehrerer Sensoren zu messen und im Sinne einer Regelung einen vorgegebenen Sollwert für die Duftstoffkonzentration einzustellen.

In jedem Fall wird die an sich bekannte Luftumwälzeinrichtung im Rahmen der Erfindung gleichsam aufgerüstet und mit einem Zusatznutzen flankiert, der darin besteht, dass durch den ergänzten Duftstoffspender die Luftumwälzeinheit der Umwälzeinrichtung nicht nur für die Luftumwälzung sorgt, sondern zugleich als Duftstoffverteiler fungiert. Hierin sind die wesentlichen Vorteile gesehen.

Im Folgenden wird die Erfindung anhand eines Ausführungsbeispiels näher erläutert; es zeigen:
- Fig. 1: die erfindungsgemäße Vorrichtung in Kombination mit einem Kühlmöbel und
- Fig. 2: eine Variante bei einem Aggregat aus Warenpräsentationsmöbel und Kühleinrichtung.

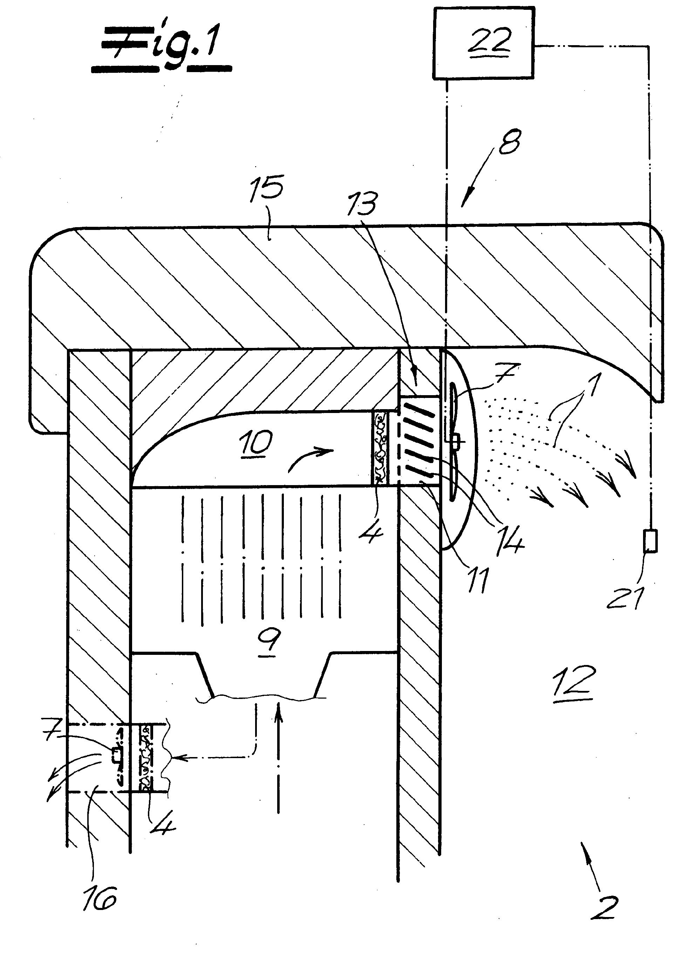

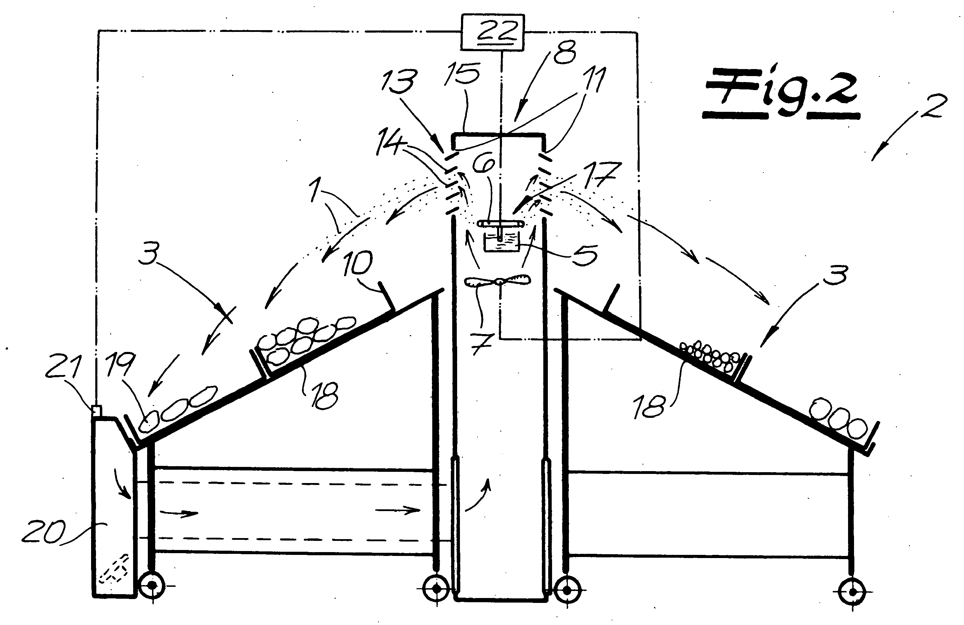

In den Figuren ist eine Vorrichtung zur Abgabe von Duftstoffen 1 dargestellt. Die Duftstoffe 1 sind lediglich in Gestalt von fein verteilten Tröpfchen 1 gezeigt und werden im Rahmen der Variante nach Fig. 1 in einen Raum, insbesondere Verkaufsraum 2, abgegeben. Bei der Ausführungsform nach Fig. 2 findet die Beduftung mit Hilfe der Duftstoffe 1 im Bereich einer Warenpräsentationseinheit bzw. eines Warenpräsentationsmöbels 3 statt. Die fragliche Vorrichtung verfügt grundsätzlich über einen Duftstoffspender 4; 5, 6 sowie einen Duftstoffverteiler 7, die nachfolgend noch näher erläutert werden.

Von besonderer Bedeutung ist der Umstand, dass eine an sich bekannte Luftumwälzeinrichtung 8 mit dem Duftstoffspender 4; 5, 6 ausgerüstet wird. Hierdurch fungiert eine Luftumwälzeinheit 7 der Luftumwälzeinrichtung 8 nicht nur als Luftumwälzer, sondern zugleich als Verteiler für die Duftstoffe 1 bzw. Duftstoffverteiler 7. Das heißt, die an sich bekannte Luftumwälzeinrichtung 8 wird in Verbindung mit dem zusätzlichen Duftstoffspender 4; 5, 6 zur gleichzeitigen Luftumwälzung und Luftbeduftung im Zuge beispielsweise der Verkaufsförderung eingesetzt. Dazu werden der Raum bzw. Verkaufsraum 2 nach der Fig. 1 und/oder die Warenpräsentationseinheit 3 im Rahmen der Variante nach Fig. 2 entsprechend beaufschlagt.

Die Luftumwälzeinrichtung 8 kann als Kühleinrichtung und/oder Klimatisierungseinheit ausgeführt sein. Bei der Variante nach Fig. 1 handelt es sich bei der Luftumwälzeinrichtung 8 um eine spezielle Kühleinrichtung, nämlich ein Kühlmöbel, welches vorliegend und nicht einschränkend als Kühlregal ausgebildet ist. Dagegen ist die Kühleinrichtung nach der Fig. 2 als transportables Kühlmöbel mit unterseitigen Rollen ausgeführt und formt in Verbindung mit der Warenpräsentationseinheit 3 ein zusammengesetztes Aggregat aus der Warenpräsentationseinheit 3 bzw. dem Warenpräsentationsmöbel und der Kühleinrichtung bzw. Luftumwälzeinrichtung 8.

Folgerichtig kann die Luftumwälzeinrichtung 8 einem zugehörigen Warenpräsentationsmöbel bzw. einer Warenpräsentationseinheit 3 zugeordnet sein und/oder in ein solches Warenpräsentationsmöbel 3 integriert werden. Dabei mag die Luftumwälzeinrichtung 8 autark ausgebildet werden, wie dies die Fig. 1 mit dem zugehörigen Kühlmöbel zeigt oder kann in das in der Fig. 2 dargestellte Aggregat aus der Luftumwälzeinrichtung 8 und beispielsweise dem Warenpräsentationsmöbel bzw. der Warenpräsentationseinheit 3 integriert werden.

Im Detail ist in der Fig. 1 der obere Bereich eines Seitenteiles des fraglichen Kühlmöbels bzw. allgemein der Luftumwälzeinrichtung 8 dargestellt. Bei dem Kühlmöbel handelt es sich - wie gesagt - um eine Kühltheke oder ein Kühlregal etc.. Man erkennt einen Luftkühler 9, welcher kopfseitig in einen Kühlluftkanal 10 übergeht. Mit Hilfe der Luftumwälzeinheit 7 wird nun Luft durch den Kühler 9 über den Kühlluftkanal 10 angesaugt und über eine Austrittsöffnung bzw. Zuluftöffnung 11 als Kühlluft respektive Zuluft in einen Warenraum 12 abgegeben. Dadurch, dass die von der Luftumwälzeinheit 7 angesaugte Luft im Bereich der Zuluftöffnung 11 zusätzlich noch durch eine Luftführungseinheit 13 mit Lamellen 14 geführt wird und zugleich eine Kopfplatte 15 des Kühlmöbels für die Luftführung sorgt, wird insgesamt ein durch Pfeile angedeuteter Kühlluftstrom bzw. -schleier dargestellt, der im Idealfall eine laminare Strömung aufweist.

In dem Kühlluftstrom, genauer im Kühlluftkanal 10, ist ein Duftstoffspender 4 angeordnet, und zwar unmittelbar vor der Luftführungseinheit 13 und dem Duftstoffverteiler 7. Bei dem Duftstoffspender 4 handelt es sich im Rahmen der Variante nach Fig. 1 um einen solchen, der mit einem porösen Trägermaterial 4 ausgerüstet ist, welches zur Aufnahme des Duftstoffes 1 dient. Tatsächlich wird der fragliche Duftstoff 1 in einzelnen Poren des Trägermaterials 4 aufgenommen und ist seinerseits flüssig oder auch fest.

Sobald die von der Luftumwälzeinheit 7 durch den Kühlluftkanal 10 angesaugte und mit Hilfe des Luftkühlers 9 abgekühlte Luft das poröse Trägermaterial 4 passiert, wird der in den Poren befindliche flüssige Duftstoff 1 mitgenommen und tritt zusammen mit der abgekühlten Luft durch die Zuluftöffnung 11 in den Warenraum 12. Dadurch wird gleichsam der ausgangsseitig der Zuluftöffnung 11 sich im Warenraum 12 bildende Kühlluftstrom bzw. Kühlluftschleier aromatisiert bzw. beduftet.

Alternativ oder zusätzlich kann der Duftstoff 1 aber auch über eine Abluftöffnung 16 in den Raum bzw. Verkaufsraum 2 eintreten. Dann wird nicht die mit Hilfe des Luftkühlers 9 gekühlte Luft als Träger einzelner Tröpfchen des Duftstoffes 1 eingesetzt, sondern vielmehr die von dem Luftkühler 9 erzeugte unvermeidbare Abluft. Diese Abluft mag über die Abluftöffnung 16 in den Verkaufsraum 2 austreten, wobei in diesem Fall wiederum eine Luftumwälzeinheit 7 für die entsprechende Luftbewegung sorgt. Dadurch, dass bei dieser alternativen Ausgestaltung der Duftstoffspender 4 in Gestalt des mit dem flüssigen Duftstoff 1 getränkten Trägermaterials 4 in Strömungsrichtung vor dem Duftstoffverteiler 7 bzw. der Luftumwälzeinheit 7 angeordnet ist, wird die durch die Abluftöffnung 16 austretende Luft entsprechend aromatisiert. Das heißt der Duftstoffspender findet sich im Bereich der Zuluftöffnung 11 und/oder der Abluftöffnung 16.

Von besonderer Bedeutung für die Erfindung ist der Umstand, dass die obligatorische Luftumwälzeinheit 7 eine gleichsam doppelte Funktion übernimmt. Denn sie sorgt einerseits dafür, dass die Abluft über die Abluftöffnung 16 respektive die Zuluft über die Zuluftöffnung 11 jeweils austreten und andererseits dafür, dass der Duftstoff 1 verteilt wird. Das heißt, Luftumwälzeinheit und Duftstoffverteiler 7 sind deckungsgleich. In diesem Zusammenhang kann sowohl die Abluft als auch die Zuluft - getrennt oder gemeinsam - als Träger des Duftstoffes 1 fungieren.

Eine alternative Ausführungsform des erfindungsgemäßen Duftstoffspenders 4; 5, 6 ist in der Fig. 2 dargestellt. Tatsächlich ist hier der Duftstoffspender 5, 6 wiederum - wie bei der Ausführungsform nach Fig. 1 - im Strom der Luftumwälzeinheit 7 angeordnet, und zwar im Bereich eines Luftauslasses bzw. vorliegend in der Nähe der Zuluftöffnung 11. Selbstverständlich könnte der dortige Duftstoffspender 5, 6 alternativ oder zusätzlich auch in der Nähe einer nicht ausdrücklich dargestellten Abluftöffnung 16 platziert werden.

Tatsächlich setzt sich der Duftstoffspender 5, 6 bei der Variante nach Fig. 2 aus einer Verteileinrichtung 6 für den Duftstoff 1 und einem Reservoir 5 für den fraglichen Duftstoff 1 zusammen. Da vorliegend flüssiger Duftstoff 1 zum Einsatz kommt, ist die Verteileinrichtung 6 als Düsenanordnung mit beidseitigen Austrittsdüsen ausgeführt, während das Reservoir 5 als Flüssigkeitsreservoir gestaltet ist. Der fragliche Duftstoffspender 5, 6 wird an dieser Stelle mit einer Befeuchtungseinheit 17 kombiniert. Tatsächlich sind die Befeuchtungseinheit 17 und der Duftstoffspender 5, 6 deckungsgleich. Das erreicht die Erfindung dadurch, dass in dem (Flüssigkeits-)Reservoir 5 ein Gemisch aus Wasser und dem Duftstoff 1 aufgenommen wird, so dass die Verteileinrichtung 6 mit ihren beidseitigen Düsen in dem angedeuteten Luftstrom ein Aerosol mit feinen Wassertröpfchen und Tröpfchen des Duftstoffes 1 erzeugt. Für die Führung des Luftstromes sorgt wiederum die Luftumwälzeinheit 7, die zugleich den Duftstoffverteiler 7 darstellt.

Man erkennt, dass bei der Variante nach Fig. 2 die Kühleinrichtung 8 mit einer Warenpräsentationseinheit bzw. einem Warenpräsentationsmöbel 3 im Sinne eines kombinierten Aggregates 3, 8 verbunden ist. Tatsächlich sind die Kühleinrichtung 8 und das Warenpräsentationsmöbel 3 jeweils modular mit einem eigenen Gestell aufgebaut und können untereinander verbunden werden. Das Warenpräsentationsmöbel 3 verfügt über eine schräg abfallende Warenauflage 18, auf der angedeutete Waren 19 platziert sind.

Die gekühlte und mit Duftstoffen 1 aromatisierte sowie gegebenenfalls befeuchtete Luft tritt über die Luftführungseinheit 13 aus jeweils beidseitig der Kühleinrichtung 8 vorgesehenen Zuluftöffnungen 11 aus. Dabei sorgen die Lamellen 14 der Luftführungseinheit 13 dafür, dass der fragliche Luftstrom im Wesentlichen parallel zur Warenauflage 18 umgelenkt wird und endseitig der Warenauflage 18 eine Rücklufteinrichtung 20 erreicht. In der Rücklufteinrichtung 20 wird der Luftstrom wiederum umgelenkt und zu der Kühleinrichtung 8 im Sinne eines Kreislaufes zurückgeführt.

In beiden Beispielfällen nach den Fig. 1 und 2 ist noch ein Sensor 21 angedeutet, mit dessen Hilfe die Konzentration der Duftstoffe 1 im Verkaufsraum 2 (Fig. 1) respektive im Bereich der Warenpräsentationseinheit bzw. des Warenpräsentationsmöbels 3 (vgl. Fig. 2) gemessen wird. Der Sensor 21 ist an eine lediglich angedeutete Steuereinheit 22 angeschlossen, die bei der Variante nach Fig. 1 die Luftumwälzeinheit bzw. den Duftstoffverteiler 7 gegebenenfalls intermittierend beaufschlagt, um die Konzentration an Duftstoffen 1 im Verkaufsraum 2 auf einen an der Steuereinheit 22 vorgewählten Wert im Sinne einer Regelung einzustellen. Bei der Ausführungsform nach Fig. 2 sorgt die mit Werten des Sensors 21 gefütterte Steuereinheit 22 dafür, dass die Düsen der Verteileinrichtung 6 (intermittierend) geöffnet und geschlossen werden. Selbstverständlich können beide Vorgehensweisen auch kombiniert werden.

Auf diese Weise kann jedenfalls die Konzentration der Duftstoffe 1 - nun im Bereich der Warenpräsentationseinheit bzw. des Warenpräsentationsmöbels 3 - auf einen vorgegebenen Wert eingestellt werden. Außerdem liegt es im Rahmen der Erfindung mit verschiedenen Duftstoffen 1 zu arbeiten. Diese können mit Hilfe der Steuereinheit 22 nicht nur in Abhängigkeit vorgegebener Konzentrationen abgegeben werden. Sondern es ist ergänzend denkbar, die Duftstoffmenge und gegebenenfalls eine Duftstoffkombination von der Jahreszeit, der Temperatur, der Uhrzeit etc. abhängig zu machen bzw. diese Parameter zur Variation zu nutzen.

## Patentansprüche

1. Vorrichtung zur Abgabe von Duftstoffen (1) an beispielsweise Verkaufsräume (2), im Bereich von Warenpräsentationsmöbeln (3) oder dergleichen, mit einem Duftstoffspender (4; 5, 6) und einem Duftstoffverteiler (7), **dadurch gekennzeichnet, dass** eine an sich bekannte Luftumwälzeinrichtung (8) mit dem Duftstoffspender (4; 5, 6) ausgerüstet wird, so dass deren Luftumwälzeinheit (7) zugleich als Duftstoffverteiler (7) fungiert.

2. Vorrichtung nach Anspruch 1, **dadurch gekennzeichnet, dass** der Duftstoffspender (4; 5, 6) mit einem Trägermaterial (4) zur Aufnahme des Duftstoffes (1) ausgebildet ist.

3. Vorrichtung nach Anspruch 2, **dadurch gekennzeichnet, dass** das Trägermaterial (4) porös ausgeführt ist und beispielsweise eine Matte, einen Filter etc. darstellt.

4. Vorrichtung nach einem der Ansprüche 1 bis 3, **dadurch gekennzeichnet, dass** der Duftstoffspender (4; 5, 6) im Luftstrom der Luftumwälzeinheit (7) angeordnet ist, und zwar vorzugsweise im Bereich eines Luftauslasses.

5. Vorrichtung nach einem der Ansprüche 1 bis 4, **dadurch gekennzeichnet, dass** der Duftstoffspender (4; 5, 6) eine Verteileinrichtung (6) für den flüssigen Duftstoff (1), beispielsweise einen Spraykopf, eine Düse, etc. aufweist.

6. Vorrichtung nach einem der Ansprüche 1 bis 5, **dadurch gekennzeichnet, dass** der Duftstoffspender (4; 5, 6) mit einer Befeuchtungseinheit (17) kombiniert wird.

7. Vorrichtung nach einem der Ansprüche 1 bis 6, **dadurch gekennzeichnet, dass** die Luftumwälzeinrichtung (8) als Kühleinrichtung (8) und/oder Klimatisierungseinheit ausgebildet ist.

8. Vorrichtung nach einem der Ansprüche 1 bis 7, **dadurch gekennzeichnet, dass** die Luftumwälzeinrichtung (8) einem Warenpräsentationsmöbel (3) zugeordnet ist und/oder in ein solches Warenpräsentationsmöbel (3) integriert ist.

9. Vorrichtung nach einem der Ansprüche 1 bis 8, **dadurch gekennzeichnet, dass** die Luftumwälzeinrichtung (8) autark ausgebildet oder in ein Aggregat (3, 8) aus der Luftumwälzeinrichtung (8) und beispielsweise einem Warenpräsentationsmöbel (3) integriert ist.

10. Verwendung einer an sich bekannten Luftumwälzeinrichtung (8) in Verbindung mit einem zusätzlichen Duftstoffspender (4; 5, 6) zur gleichzeitigen Luftumwälzung und Luftbeduftung im Zuge der Verkaufsförderung.

## Geänderte Patentansprüche

### Geänderte Patentansprüche gemäss Regel 137(2) EPÜ.

**1.** Verwendung einer Vorrichtung zur Abgabe von Duftstoffen (1) an Verkaufsräume (2) im Bereich von Warenpräsentationsmöbeln (3) im Zuge der Verkaufsförderung, mit einem Duftstoffspender (4; 5, 6) und einem Duftstoffverteiler (7), wobei
- eine an sich bekannte Luftumwälzeinrichtung (8) mit dem Duftstoffspender (4; 5, 6) ausgerüstet wird, sodass deren Luftumwälzeinheit (7) zugleich als Duftstoffverteiler (7) fungiert, und wobei
- der Duftstoffspender (4; 5, 6) in bestimmten Zeitabständen getaktet betrieben wird, um die Konzentration des einen oder der mehreren Duftstoffe im Verkaufsraum gezielt zu variieren und für die entsprechende Verkaufsförderung zu nutzen.

**2.** Verwendung nach Anspruch 1, **dadurch gekennzeichnet, dass** der Duftstoffspender (4; 5, 6) mit einem Trägermaterial (4) zur Aufnahme des Duftstoffes (1) ausgebildet ist.

**3.** Verwendung nach Anspruch 2, **dadurch gekennzeichnet, dass** das Trägermaterial (4) porös ausgeführt ist und beispielsweise eine Matte, einen Filter etc. darstellt.

**4.** Verwendung nach einem der Ansprüche 1 bis 3, **dadurch gekennzeichnet, dass** der Duftspender (4; 5, 6) im Luftstrom der Luftumwälzeinheit (7) angeordnet ist, und zwar vorzugsweise im Bereich eines Luftauslasses.

**5.** Verwendung nach einem der Ansprüche 1 bis 4, **dadurch gekennzeichnet, dass** der Duftstoffspender (4; 5, 6) eine Verteileinrichtung (6) für den flüssigen Duftstoff (1), beispielsweise einen Spraykopf, eine Düse, etc. aufweist.

**6.** Verwendung nach einem der Ansprüche 1 bis 5, **dadurch gekennzeichnet, dass** der Duftstoffspender (4; 5, 6) mit einer Befeuchtungseinheit (17) kombiniert wird.

**7.** Verwendung nach einem der Ansprüche 1 bis 6, **dadurch gekennzeichnet, dass** die Luftumwälzeinrichtung (8) als Kühleinrichtung (8) und/oder Klimatisierungseinheit ausgebildet ist.

**8.** Verwendung nach einem der Ansprüche 1 bis 7, **dadurch gekennzeichnet, dass** die Luftumwälzeinrichtung (8) einem Warenpräsentationsmöbel (3) zugeordnet ist und/oder in ein solches Warenpräsentationsmöbel (3) integriert ist.

**9.** Verwendung nach einem der Ansprüche 1 bis 8, **dadurch gekennzeichnet, dass** die Luftumwälzeinrichtung (8) autark ausgebildet oder in ein Aggregat (3, 8) aus der Luftumwälzeinrichtung (8) und beispielsweise einem Warenpräsentationsmöbel (3) integriert ist.
